# EUROPEAN PATENT APPLICATION

(11) **EP 2 412 345 A1**
(43) Date of publication of application: **01.02.2012**
(21) Application number: 10755781.1
(22) Date of filing: 15.02.2010
(51) Int. Cl.: A61F 13/15, A61F 13/49, A61F 13/494, A61F 13/511

(54) **ABSORPTIVE ARTICLE**

(30) Priority: 25.03.2009 JP 2009075136
(71) Applicant: Unicharm Corporation, Ehime 799-0111 (JP)
(72) Inventor: YAMANAKA, Yasuhiro, Kanonji-shi Kagawa 769-1602 (JP); WAKASUGI, Kei, Kanonji-shi Kagawa 769-1602 (JP); YAGO, Toshiya, Kanonji-shi Kagawa 769-1602 (JP); OHASHI, Naoto, Kanonji-shi Kagawa 769-1602 (JP)
(74) Representative: Eke, Philippa Dianne
(86) International application number: PCT/JP2010/052223
(87) International publication number: WO 2010/109980

(57) **Abstract**

The present invention provides an absorbent article adapted to prevent body waste from leaking beyond leakage-barrier cuffs and thereby to restrict leakage of body waste from the article. A chassis 14 forming a diaper 10 includes a top-sheet 40 lying on a side facing the wearer's skin and a back-sheet 50 lying on the opposite side wherein the top-sheet 40 has a plurality of ridges extending in a longitudinal direction Y and a plurality of grooves each defined between a pair of the adjacent ridges. Opposite side edges 23 of a liquid-absorbent structure 20 respectively include middle segments extending in a crotch region 13 and front and rear segments respectively extending in front and rear waist regions 11, 12 having width dimensions in a transverse direction X larger than a width dimension in the transverse direction of the middle segments. Outboard of the middle segments, imaginary lines 90 respectively connecting the front segments to the rear segments cooperate with the side edges 23 of the liquid-absorbent structure to define absorbent structure-absent zones 91. Leakage-barrier cuffs 30 are attached to the side of the top-sheet 40 so that the absorbent structure-absent zones 91 may extend between proximal side edges 34 of the cuffs and the side edges 23 of the liquid-absorbent structure.

## Description

### {Technical Field}

The present invention relates to absorbent articles and, more particularly to absorbent articles as disposable diapers, toilet-training pants or incontinent briefs.

### {Background}

Disposable diapers including gather sheets adapted to raise themselves against the wearer's skin as the diaper is put on the wearer's body is known, for example, from the disclosure of JP 2007-143874 A (PTL 1). This known diaper includes top- and back-sheets, an absorbent structure sandwiched between these top- and back-sheets, a pair of gather sheets formed on a side of the top-sheet facing the wearer's skin. These gather sheets may raise themselves so as to be spaced upward from the top-sheet and thereby form barriers against body wastes to prevent the body wastes from leaking out from the diaper. The absorbent structure is shaped just like the crotch region, i.e., a width dimension relatively small in its middle zone and relatively large in front and rear ends.

### {Citation List}

### {Patent Literature}

{PTL 1} JP 2007-143874 A

### {Summary}

### {Technical Problem}

In the known diaper as has been described above, the gather sheets are bonded to the top-sheet completely overlapping the absorbent structure having a relatively large width dimension in vicinities of the front and rear ends thereof. However, in a vicinity of the middle zone thereof, the gather sheets are bonded to the top-sheet outboard of the absorbent structure as viewed in the transverse direction since the width dimension of the absorbent structure is relatively small in the vicinity of its middle zone. As a consequence, between the front and rear ends of the absorbent structure, laminate zones of only the top- and back-sheets sheet are formed. These laminate zones of only the top- and back-sheets extending outboard of the middle zone of the absorbent structure also raise themselves from the side edges of the absorbent structure as the gather sheets are spaced upward from the top-sheet. Thus, outboard of the middle zone of the absorbent structure, the top- and back-sheets raise themselves together with the gather sheets to form barriers against body waste such as urine. However, the top-sheet made of a liquid-pervious non-woven fabric as well as the back-sheet formed of a laminate of a fibrous non-woven fabric and a liquid-impervious film usually have a stiffness lower than that of the absorbent structure. In view of this, the regions defined by the laminate of only the top- and back-sheets are apt to lose initial shapes thereof even under a slight external pressure exerted thereon and eventually the gather sheets may often collapsed. The collapse of the gather sheets may cause body wastes to move outward beyond the gather sheets and to leak out from the diaper.

An object of one or more embodiments according to the present invention is to provide an absorbent article adapted to prevent body wastes from leaking beyond leakage-barrier cuffs and thereby to restrict leakage of body wastes from the article.

### {Solution to Problem}

According to the present invention, there is provided an absorbent article having a longitudinal direction and a transverse direction, including a chassis including a side facing the wearer's skin, a side opposite thereto, a front waist region, a rear waist region and a crotch region extending between the front and rear waist regions; a liquid-absorbent structure extending at least across the crotch region; and a pair of leakage-barrier cuffs provided on the side of the chassis facing the wearer's skin and extending along respective side edges of the chassis in the longitudinal direction, wherein the chassis further includes a top-sheet lying on the side facing the wearer's skin and a back-sheet lying on the opposite side; the leakage-barrier cuffs respectively including outer side edges bonded to associated side edges of the chassis by an intermediary of associated side edge bonding zones and, inner side edges contractibly elasticized in the longitudinal direction so that the inner side edges are spaced upward from the chassis; the liquid-absorbent structure has a pair of side edges extending in the longitudinal direction each including a middle segment which extends in the crotch region and has a relatively small length dimension in the transverse direction and front and rear segments which extend in the front and rear waist regions, respectively, and having length dimensions larger than that of the middle segment; and the outer side edges of the leakage-barrier cuffs lie outboard of the middle segment as viewed in the transverse direction.

The aforementioned one or more embodiments of the present invention further includes the top-sheet being formed at least outboard of the middle segment as viewed in the transverse direction with a plurality of ridges and a plurality of grooves each formed between each pair of the ridges adjacent in the transverse direction and bonded to the back-sheet.

According to the aforementioned one or more embodiments of the present invention, the ridges as well as the grooves are formed on the side of the top-sheet facing the wearer's skin.
According to the aforementioned one or more embodiments of the present invention, the outer side edges of the cuffs are bonded to the side of the top-sheet facing the wearer's skin.
According to the aforementioned one or more embodiments of the present invention, the outer side edges of the cuffs are bonded to the side of the back-sheet facing the wearer's skin, and the top-sheet includes opposite side edges extending in the longitudinal direction and the opposite side edges are bonded to the inner side edges of the cuffs.

### {Advantageous Effects of Invention}

The absorbent article includes the top- and back-sheets, the liquid-absorbent structure sandwiched between these top- and back-sheets and the leakage-barrier cuffs provided on the side of the top-sheet facing the wearer's skin. The liquid-absorbent structure includes the middle segment having a relatively small transverse width dimension and the front and rear regions each having a transverse width dimension larger than that of the middle region. Outboard of the middle region, the outer side edges of the respective leakage-barrier cuffs are attached to the chassis. The absorbent structure-absent zones defined between the middle segment of the absorbent structure and the leakage-barrier cuffs also flex upward when the leakage-barrier cuffs raise themselves from the top-sheet toward the wearer's skin. The top-sheet is formed with a plurality of ridges and the grooves extending in the longitudinal direction so that the transverse stiffness thereof is effectively enhanced and consequently the top-sheet is resistant to flexion in the direction orthogonal to the convex and grooves. Such high transverse stiffness allows the absorbent structure-absent zones defined outboard of side edge of the middle segment of the absorbent structure to maintain the initial shapes of these absorbent structure-absent zones and thereby to prevent leakage of body waste due to collapsing and/or deformation of the leakage-barrier cuffs. In this way, leakage of body wastes from the absorbent article can be reliably restricted.

### {Brief Description of Drawings}

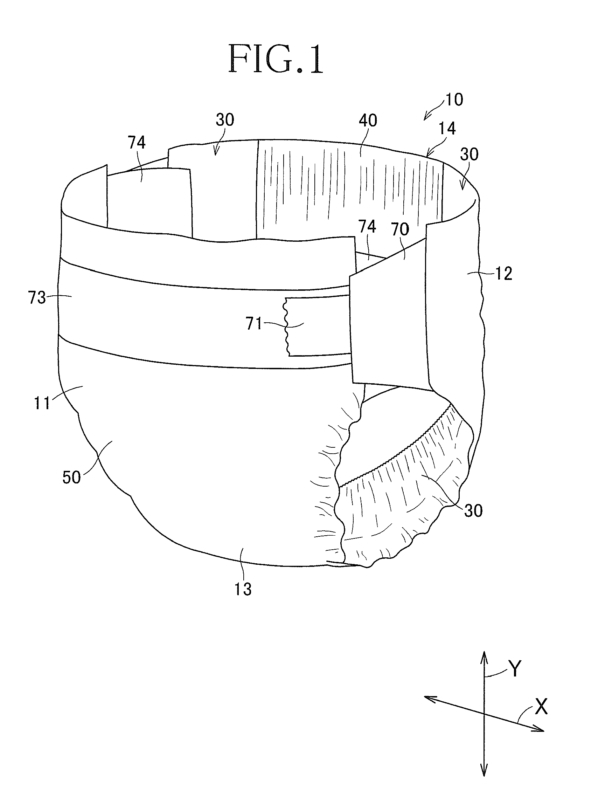

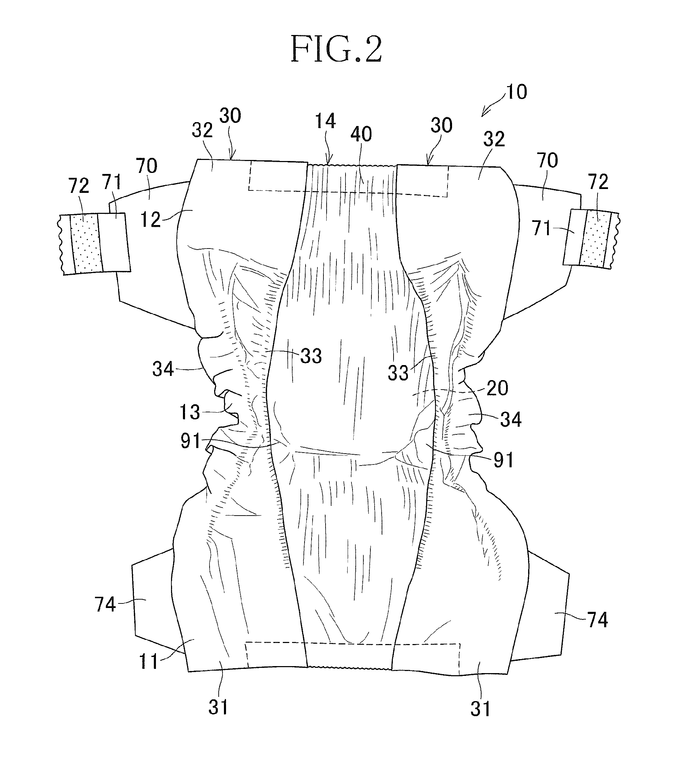

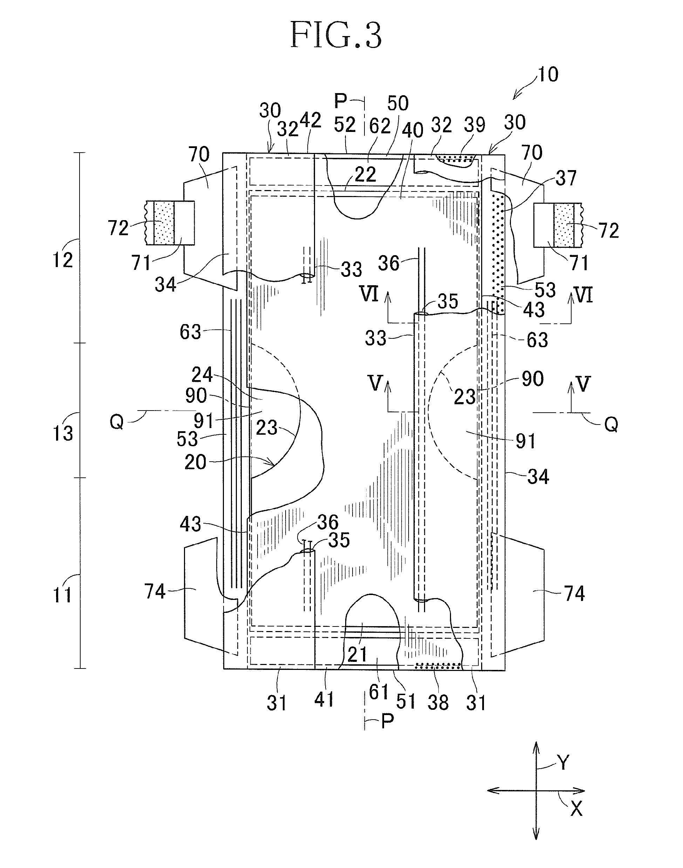

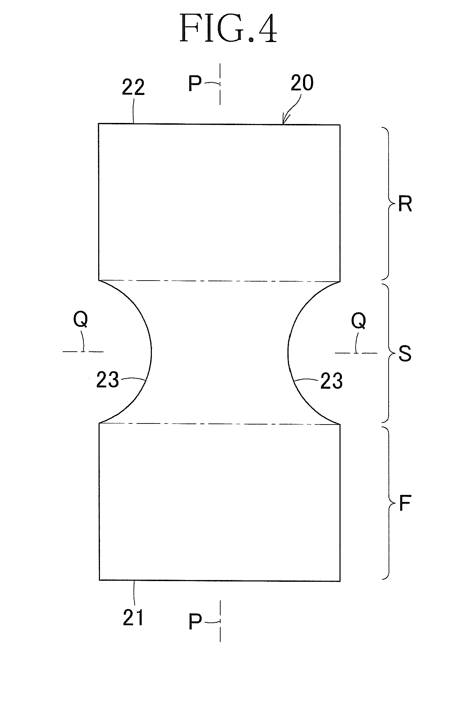

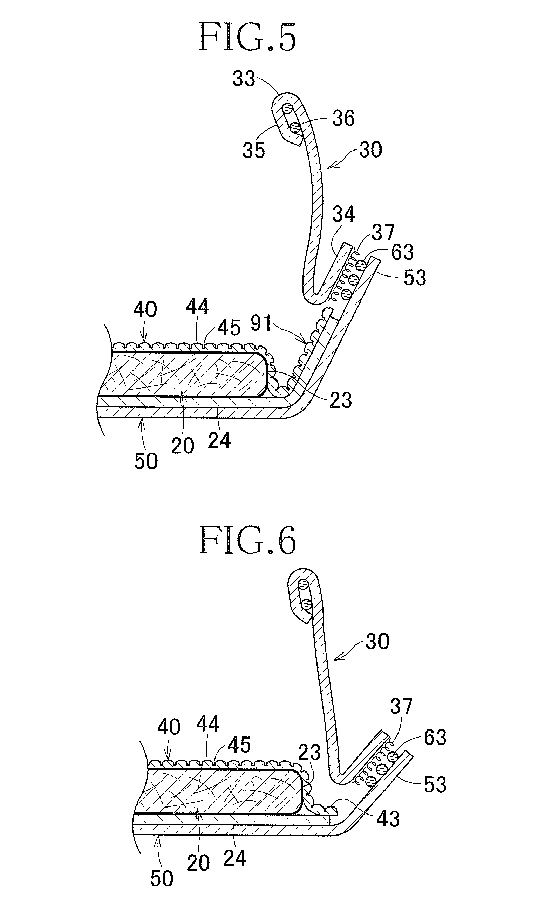

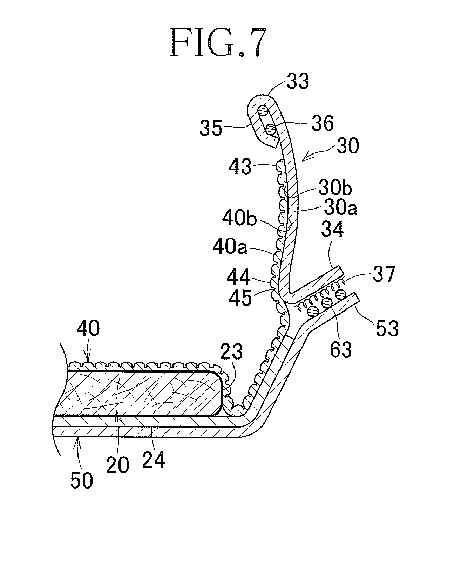

{Fig. 1} A perspective view of a disposable diaper according to a first exemplary embodiment of the invention in its state as put on the wearer's body.
{Fig. 2} An open view of the diaper of Fig. 1 with elastic members not stretched.
{Fig. 3} A plan view of the diaper of Fig. 2 opened flat by stretching respective elastic members against a contractile force thereof.
{Fig. 4} A plan view of a liquid-absorbent structure.
{Fig. 5} A sectional view taken along the line V-V in Fig. 3.
{Fig. 6} A sectional view taken along the line VI-VI in Fig. 3.
{Fig. 7} A view similar to Fig. 5, of a diaper according to a second exemplary embodiment.

### {Description of Embodiments}

Details of an absorbent article according to the present invention will be more fully understood from the description of a disposable diaper as exemplary embodiments of the absorbent article given hereunder with reference to the accompanying drawings.

### {Embodiment 1}

Figs. 1 through 6 illustrate a first exemplary embodiment of the present invention wherein Fig. 1 is a perspective view of a diaper 10 according to the first exemplary embodiment of the invention in a state put on the wearer's body, Fig. 2 is an open view of the diaper 10 after fastening members in front and rear waist regions have been unfastened from each other, Fig. 3 is a plan view of this diaper 10 opened flat by stretching respective elastic members against a contractile force thereof, Fig. 4 is a plan view of a liquid-absorbent structure 20 in Fig. 3, Fig. 5 is a sectional view taken along the line V-V in Fig. 3, and Fig. 6 is a sectional view taken along the line VI-VI in Fig. 3. Fig. 2 is partially cutaway for convenience of illustration. Figs. 5 and 6 illustrate behavior of a leakage-barrier cuff under the contractile force of the associated elastic members.

The diaper 10 basically includes a chassis 14, a liquid-absorbent structure 20 and a pair of leak-barrier cuffs 30 provided on a side of the chassis 14 facing the wearer's skin. The diaper 10 has a longitudinal direction Y and a transverse direction X. The chassis 14 includes the side facing the wearer's skin, a side facing the wearer's garments, a front waist region 11, a rear waist region 12 and a crotch region 13 extending between these front and rear waist regions 11, 12 as viewed in the longitudinal direction Y. The liquid-absorbent structure 20 extends across the crotch region 13 into the front and rear waist regions 11, 12. The diaper 10 has an imaginary longitudinal center line P-P bisecting a dimension of the diaper 10 in the transverse direction X and an imaginary transverse center line Q-Q bisecting a dimension of the diaper 10 in the longitudinal direction Y. The diaper 10 is shaped to be substantially symmetric about the imaginary longitudinal center line P-P.

The chassis 14 further includes a top-sheet 40 facing the wearer's skin and a back-sheet 50 facing away from the wearer's skin. Referring to Fig. 3, the top- and back-sheets 40, 50 respectively have front and rear ends 41, 42; 51, 52 extending in the transverse direction X and side edges 43, 43; 53, 53 extending in the longitudinal direction Y wherein a length dimension of the top-sheet 40 in the transverse direction X is smaller than that of the back-sheet 50 so that the side edges 53, 53 of the back-sheet 50 extend outward beyond the side edges 43, 43 of the top-sheet 40 and define side edges of the chassis 14. Front and rear ends 41, 42 of the top-sheet 40 substantially correspond to front and rear ends 51, 52 of the back-sheet 50 in the positional relationship so that these ends define front and rear ends of the chassis 14.

The rear waist region 12 of the back-sheet 50 is provided with rear side sheets 70 extending outward in the transverse direction X from the respective side edges 53 and these rear side sheets 70, 70 are respectively formed, in turn, with hook elements 72 by an intermediary of tab members 71. The front waist region 11 of the back-sheet 50 is provided with front side sheets 74 extending outward in the transverse direction X from the respective side edges 53 and these front side sheets 74 are provided on the respective sides facing the wearer's garments with loop elements 73 (See Fig. 1) extending in the transverse direction X on the inward side of the respective front side sheets 74. The hook elements 72 are adapted to be engaged with the associated loop elements 73 and thereby to form the pants-type diaper 10.

Referring to Figs. 5 and 6, the top-sheet 40 has a plurality of ridges 44 extending n the longitudinal direction Y and a plurality of grooves 45 each extending in the longitudinal direction Y between each pair of the adjacent ridges 44. A thickness (i.e., a three dimensional direction) dimension of the ridge 44 may be in a range of about 1. 0 to 3.0mm and about 1. 3mm in this embodiment. A thickness (i.e., a three dimensional direction) dimension of the groove 45 may be in a range of about 0.4 to 1.5mm and about 0.75mm in this embodiment. These thickness dimensions were measured by using KEYENCE's laser displacement gauge (KEYENCE LK-G30). Specifically, a table supporting the top-sheet 40 was placed at a distance of 30.5cm from the laser irradiation hole and the measurement was measured over a range of 26mm along the ridge 44 and the groove 45. As used here, the term "thickness dimension of the ridge 44" refers to an average value of length dimensions from the table to an apex of crest defined by the ridge 44 measured over the range of 26mm, and also as used here, the term "thickness dimension of the groove 45" refers to an average value of depth dimensions to a bottom of trough defined by the groove 45 measured over the range of 26mm. A pitch corresponding to a length dimension between a pair of the adjacent ridges 44 was about 4mm.

Such top-sheet 40 can be easily folded along the grooves 45, i.e., in the longitudinal direction but not orthogonally to the grooves 45, i.e., in the transverse direction. The stiffness against which the top-sheet 40 can be folded along the ridges 44 and the grooves 45 is referred to as the longitudinal stiffness and the stiffness against which the top-sheet 40 can be folded orthogonally to the ridges 44 and the grooves 44, 45 is referred to as the transverse stiffness. The longitudinal stiffness may be in a range of about 100mm to about 140mm and is preferably about 120mm in this embodiment. The transverse stiffness may be in a range of about 50mm to about 90mm and is preferably about 70mm in this embodiment. The longitudinal stiffness is preferably about 1.7 times of the transverse stiffness. These stiffness values were measured in accordance with JIS L1018 8. 22. 1 A.

The chassis 14 is provided along the front and rear ends thereof with front and rear waist elastic members 61, 62 attached under tension thereto in a contractible manner. The front and rear waist elastic elements 61, 62 are preferably formed of a foamed polyurethane sheet containing therein open cells and bonded to at least one of the top- and back-sheets 40, 50 by means of bonding means such as hot melt adhesive (not shown). The top-sheet 40 is easily contracted in the transverse direction X since its longitudinal stiffness along the ridges 44 and the grooves 45 is relatively low and therefore the top-sheet 40 should not noticeably affect contraction of these front and rear waist elastic bands 61, 62.

The chassis 14 is provided along the respective side edges with associated leg elastic elements 63 extending in the longitudinal direction Y attached thereto in a contractible manner. Each of the leg elastic elements 63 is formed of a plurality of elastic threads or yarns and attached between the back-sheet 50 and the leakage-barrier cuff 30. The leg elastic elements 63 are attached to the chassis 14 outboard of the top-sheet 40 as viewed in the transverse direction X and therefore the top-sheet 40 having the relatively high transverse stiffness in the direction orthogonal to the ridges 44 and the grooves 45 should not affect contraction of these leg elastic elements 63.

The liquid-absorbent structure 20 is sandwiched between the top- and back-sheets 40, 50. The liquid-absorbent structure 20 has front and rear ends 21, 22 extending in the transverse direction X and side edges 23, 23 extending in the longitudinal direction Y. The front end 21 is located in the front waist region 11 and attached to the chassis substantially adjacent to the rear waist elastic element 62 as viewed in the longitudinal direction Y.

As will be apparent from Fig. 4, the side edges 23 of the liquid-absorbent structure 20 respectively include a middle segment S curving in the crotch region 13 toward the imaginary longitudinal center line P-P to reduce the width-dimension of the liquid-absorbent structure 20 in the transverse direction X and front and rear segments F, R extending in the front and rear waist regions with the width-dimension of the liquid-absorbent structure 20 in the transverse direction X being larger than the width-dimension of the structure in the crotch region 13. The minimum width-dimension in the transverse direction X between opposite side edges of the middle segment S is approximately in a range of 70 to 90mm as measured along the imaginary transverse center line Q-Q and the width-dimension between opposite side edges of the front segment F as well as the width-dimension between opposite side edges of the rear segment R respectively is approximately 130mm. Outboard of the middle segment S in the transverse direction X, imaginary lines 90 connecting side edges of the front segment F to side edges of the rear segment R cooperate with the associated concave side edges of the middle segment S to define absorbent structure-absent zones 91, respectively.
The liquid-absorbent structure 20 includes, for example, a liquid-absorbent core containing a mixture of fluff pulp fibers and super-absorbent polymer particles, and a liquid-dispersant sheet such as tissue paper with which the liquid-absorbent core is wrapped. A liquid-impervious leakage-barrier sheet such as a film is sandwiched between the liquid-absorbent structure 20 and the back-sheet 50.

The leakage-barrier cuffs 30 attached to the side of the top-sheet 40 facing the wearer's skin have front and rear ends 31, 32 opposite to each other in the longitudinal direction Y and distal and proximal side edges 33, 34 extending in the longitudinal direction Y. The front and rear ends 31, 32 substantially correspond to the front and rear ends 41, 42 of the top-sheet 40 in positional relationship and bonded to these front and rear ends 41, 42 of the top-sheet 40 at bonding zones 38, 39. The proximal side edges 34 of the cuffs 30 are bonded to the chassis 14 at side edge bonding zones 37. It should be appreciated here that these side edge bonding zones 37 are defined between the proximal side edges 34 of the respective cuffs 30 and the respective side edges 53 of the back-sheet 50 extending outward beyond the respective side edges of the top-sheet 40. Leg elastic elements 63 are attached along these side edge bonding zones 37 between the proximal side edges 34 and the outer side edges 53 of the back-sheet 50.

The distal side edges 33 of the cuffs 30 are partially folded back onto the top-sheet 40 so as to form respective sleeves 35 within which cuff-biasing elastic elements 36 extending in the longitudinal direction Y are attached under tension. Each of the cuff-biasing elastic element 36 includes a plurality of elastic threads or yarns and is attached under tension within the associated sleeve 35 at a predetermined distance from the front and rear ends 31, 32 of this cuff 30 so that the leakage-barrier cuffs 30 may be elasticized in the longitudinal direction Y at least in the crotch region 13. Alternatively, an elasticized non-woven fabric or the like may be attached to the distal side edges 33 of the respective cuffs 30 and thereby the leakage-barrier cuffs 30 may be elasticized in the longitudinal direction Y.

In response to contraction of the cuff-biasing elastic elements 36 in the diaper 10 as illustrated in Fig. 2, the distal side edges 33 of the respective cuffs 30 are spaced upward from the top-sheet 40 as if they rise thereon. At the same time, in the front and rear waist regions 11, 12, the proximal side edges 34 of the cuffs 30 extending close to the front and rear zones F, R of the liquid-absorbent structure 20 and attached to the chassis 14 outboard of the liquid-absorbent structure 20 as viewed in the transverse direction X function to move the front and rear waist regions 11, 12 closer to each other toward the wearer's body. As illustrated in Fig. 5, the absorbent structure-absent zones 91 are defined in the crotch region between the proximal side edges 34 of the respective cuffs 30 and the respective side edges 23 of the liquid-absorbent structure 20. These absorbent structure-absent zones 91 also are pulled in close contact with the wearer's body by an intermediary of the proximal side edges 34 of the respective cuffs 30.

The absorbent structure-absent zones 91 are free from any section of the liquid-absorbent structure 20 and defined by the top- and back-sheets 40, 50. This means that these absorbent structure-absent zones 91 are correspondingly less stiff and can be smoothly flexed along the liquid-absorbent structure 20. Specifically, the longitudinal stiffness of the top-sheet 40 is relatively low because of the presence of the ridges 44 and the grooves 45 and therefore the top-sheet 40 can be easily flexed upward about the respective side edges 23 of the liquid-absorbent structure 20. In the crotch region 13, consequentially, the leakage-barrier cuffs 30 can be flexed upward into close contact with the wearer's skin over the dimension thereof in the transverse direction X inclusive of the absorbent structure-absent zones 91 to form high barriers in close contact with the wearer's skin. The region in the diaper 10 which is at the highest risk of body waste (e.g., urine) leakage is a pair of leg-openings since these leg-openings are significantly affected by the movement of the wearer. However, the present invention allows the cuffs 30 to form high leakage-barriers in the crotch region 13 defining the leg-openings and thereby to prevent any body waste leakage in an efficient manner.

With the diaper 10 put on the wearer's body, even if the leakage-barrier cuffs 30 are subject to any pressure such as body weight, the absorbent structure-absent zones 91 flex so as to shrink along the ridges 44 and the grooves 44 of the top-sheet 40 and to buffer the body weight. In this way, the leakage-barrier cuffs 30 can be prevented from being collapsed. In addition, a relatively high resistance of the top-sheet 40 to flexion in the direction orthogonal to the ridges 44 and the grooves 45 makes it difficult for the leakage-barrier cuffs 30 inclusive of the respective absorbent structure-absent zones 91 to be deformed in the longitudinal direction Y. More specifically, while the wearer's inguinal region behaves to tuck the absorbent structure-absent zones 91 inward to the imaginary longitudinal center line P-P, the high resistance of the top-sheet 40 to flexion thereof in the direction orthogonal to the ridges 44 and the grooves 45 advantageously makes such tucking-in of the absorbent structure-absent zones 91 toward the imaginary longitudinal center line P-P. In a consequence, the shapes of the inner side edges 33 of the respective cuffs 30 can be maintained. In this way, body waste such as urine can be reliably prevented by the leakage-barrier cuffs 30 from leaking out from the diaper 10.

The front and rear waist elastic elements 61, 62 are attached under tension, i.e., contractibly, to the chassis 14 between the top- and back-sheets 40, 50 so that the top-sheet 40 also may contract in the transverse direction X as these front and rear waist elastic elements 61, 62 contract. Upon contraction of the top-sheet 40 in the transverse direction X, the ridges 44 move closer into contact one with another so as to reduce a dimension of each groove 45 substantially to zero. As an advantageous result, a clearance inevitably defined between the top-sheet 40 and the wearer's skin can be reduced and thereby leakage of body waste through the waist-opening formed by the front and rear ends of the top-sheet 40 can be restricted.

The top-sheet 40 can be formed with the grooves 45 and the ridges 44, for example, by treating a fibrous web with air jet streams continuously discharged from nozzles array wherein regions of the fibrous web directly subjected to the air jet streams form the grooves 45 and regions not directly subjected to the air jet streams form the ridges 44. In such top-sheet 40, component fibers of the fibrous web may be reoriented to obtain the grooves 45 each having a density lower than that of the ridges 44. The grooves 45 can facilitate loose waste material to pass through these grooves 45 to the liquid-absorbent structure 30. Air jet quantity may be intermittently increased to form the grooves 45 with intermittent through-holes. These through-holes can serve to facilitate passage of soft feces of relatively high viscosity.

While this embodiment supposes that the ridges 44 as well as the grooves 45 of the top-sheet 40 are formed by the air jet treatment method, the invention is not limited to this process so far as the sufficient level of stiffness to maintain the initial shape of the leakage-barrier cuff 30. As an alternative method to such air jet treatment method, the top-sheet 40 may be formed with the ridges 44 and the grooves 45 also by a water jet treatment, a steam jet treatment, a press working or gear working treatment. Furthermore, it should be appreciated that the thickness dimension and the pitch of the ridges 44 as well as those of the grooves 45 are not limited to the values as indicated for this first embodiment.

### {Embodiments 2}

Fig. 7 is a view similar to Fig. 5 relating to the first exemplary embodiment, illustrating a second exemplary embodiment of the invention. This embodiment 2 is characterized in a position at which each of the leakage-barrier cuffs 30 is bonded to the top-sheet 40 and the other construction is substantially the same as that in the embodiment 1. Details of the components common to those in the embodiment 1 will not be described.

The proximal side edges 34 of the respective leakage-barrier cuffs 30 are bonded to the back-sheet 50 by a intermediary of the side edge bonding zones 37 extending in the longitudinal direction Y. The top-sheet 40 has a first surface 40a facing the wearer's skin and a second surface 40b facing the back-sheet 50. Similarly, each of the leakage-barrier cuffs 30 has a first surface 30a facing the wearer's skin and a second surface 30b opposite to the first surface 30a. The second surfaces 40a of the respective side edges 43 of the top-sheet 40 are bonded to the second surfaces 30b of the distal side edges 33 of the respective leakage-barrier cuffs 30.

The top-sheet 40 is laminated on the respective cuffs 30 along the respective distal side edges 33 and to increase stiffness of the distal side edges 33 of the respective cuffs 30 so that the distal side edges 33 thereof can maintain the initial shapes thereof even if any pressure such as body weight is exerted thereon. Particularly, the top-sheet 40 formed with the ridges 44 and the grooves 45 extending in the longitudinal direction Y is well resistant to flexion in the direction orthogonal to the these ridges 44 and the grooves 45 and thereby maintains the initial shape thereof. The top-sheet 40 is capable of coming in close contact with the wearer's skin without leaving an undesirable clearance between the top-sheet 40 and the wearer's skin. Consequentially, leakage of body waste which should otherwise occur from the crotch region 13 can be further reliably prevented.

While the side of the top-sheet 40 facing the wearer's skin is formed with the ridges 44 and the grooves 45 according to the aforementioned embodiments of the invention, it is possible to form the top-sheet 40 on the side facing the wearer's clothes with such streaks 44, 45. However, considering various factors such as the area over which the top-sheet 40 comes in contact with the wearer's skin and the permeability for bodily fluids, these streaks are preferably formed on the side of the top-sheet facing the wearer's skin. Finally, while the absorbent article has been exemplarily described above on the basis of the so-called open-type diaper, the invention is not limited to this and applicable also to so-called pull-on pants.

### {Reference Signs List}

- 10: diaper (absorbent article)
- 11: front waist region
- 12: rear waist region
- 13: crotch region
- 14: chassis
- 20: liquid-absorbent structure
- 23: side edges of liquid-absorbent structure
- 30: leakage-barrier cuffs
- 31: front end of cuffs
- 32: rear end of cuffs
- 33: distal side edges of cuffs
- 34: proximal side edges of cuffs
- 36: cuff-biasing elastic member
- 40: top-sheet
- 44: ridges
- 45: grooves
- 50: back-sheet
- 91: absorbent structure-absent zone
- S: middle segment
- F: front segment
- R: rear segment

## Claims

1. An absorbent article having a longitudinal direction and a transverse direction, comprising a chassis including a side facing the wearer's skin, a side opposite thereto, a front waist region, a rear waist region and a crotch region extending between the front and rear waist regions; a liquid-absorbent structure extending at least across the crotch region; and a pair of leakage-barrier cuffs provided on the side of the chassis facing the wearer's skin and extending along respective side edges of the chassis in the longitudinal direction, wherein the chassis further includes a top-sheet lying on the side facing the wearer's skin and a back-sheet lying on the opposite side; the leakage-barrier cuffs respectively including proximal side edges bonded to the associated side edges of the chassis by an intermediary of the associated side edge bonding zones and distal side edges contractibly elasticized in the longitudinal direction so that the distal side edges are spaced upward from the chassis; the liquid-absorbent structure having a pair of side edges extending in the longitudinal direction each including a middle segment extending in the crotch region having a relatively small length dimension in the transverse direction and front and rear segments extending in the front and rear waist regions and having length dimensions larger than the length dimension of the middle segments; and the proximal side edges of the leakage-barrier cuffs lie outboard of the middle segment as viewed in the transverse direction, the absorbent article being **characterized in that**:
the top-sheet is formed at least outboard of the middle segments as viewed in the transverse direction with a plurality of ridges and a plurality of grooves each formed between each pair of the ridges adjacent in the transverse direction and bonded to the back-sheet.

2. The absorbent article defined by Claim 1, wherein the ridges as well as the grooves are formed on the side of the top-sheet facing the wearer's skin.

3. The absorbent article defined by Claim 1 or 2, wherein the proximal side edges of the cuffs are bonded to the side of the top-sheet facing the wearer's skin.

4. The absorbent article defined by Claim 1 or 2, wherein:
the proximal side edges of the cuffs are bonded to the side of the back-sheet facing the wearer's skin; and
the top-sheet includes opposite side edges extending in the longitudinal direction and the opposite side edges are bonded to the distal side edges of the cuffs.
